(19) Europäisches Patentamt
European Patent Office
Office européen des brevets

(11) **EP 4 674 511 A1**

(12) **EUROPEAN PATENT APPLICATION**
published in accordance with Art. 153(4) EPC

(43) Date of publication:
**07.01.2026 Bulletin 2026/02**

(21) Application number: 24780451.1

(22) Date of filing: **27.03.2024**

(51) International Patent Classification (IPC):
**B01D 53/22** (2006.01)    **B01D 61/58** (2006.01)
**B01D 69/00** (2006.01)    **B01D 69/10** (2006.01)
**B01D 69/12** (2006.01)    **B01D 71/70** (2006.01)
**C07C 17/389** (2006.01)   **C07C 19/08** (2006.01)

(52) Cooperative Patent Classification (CPC):
**B01D 53/22; B01D 61/58; B01D 69/00;**
**B01D 69/10; B01D 69/12; B01D 71/70;**
**C07C 17/389; C07C 19/08**

(86) International application number:
**PCT/JP2024/012254**

(87) International publication number:
**WO 2024/204348 (03.10.2024 Gazette 2024/40)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **31.03.2023 JP 2023058564**

(71) Applicant: **Daikin Industries, Ltd.**
**Osaka-shi, Osaka 530-0001 (JP)**

(72) Inventors:
• **YAMAGUCHI, Shuhei**
  **Osaka-Shi, Osaka 530-0001 (JP)**
• **NAMIKAWA, Takashi**
  **Osaka-Shi, Osaka 530-0001 (JP)**
• **YAMAUCHI, Akiyoshi**
  **Osaka-Shi, Osaka 530-0001 (JP)**
• **KISHIKAWA, Yosuke**
  **Osaka-Shi, Osaka 530-0001 (JP)**

(74) Representative: **Hoffmann Eitle**
**Patent- und Rechtsanwälte PartmbB**
**Arabellastraße 30**
**81925 München (DE)**

(54) **GAS ISOLATION METHOD**

(57) The present disclosure aims to provide a novel method for separating a fluorocarbon.

A method for separating a fluorocarbon gas comprising:
feeding a mixed gas comprising two or more kinds of fluorocarbon gases having different molecular diameters from each other to a porous membrane and separating a gas composition in which a mixing ratio of one kind of a fluorocarbon gas selected from the two or more kinds of fluorocarbon gases is increased or a single gas, wherein the porous membrane comprises amorphous silica formed by a sol-gel method.

Fig. 1

EP 4 674 511 A1

**Description**

Technical Field

The present disclosure relates to a method for separating gas and a separation apparatus.

Background Art

Distillation of a mixture of fluorocarbons fluorocarbons has been known as a method of separating a fluorocarbon. Patent Literature 1 describes that a mixture of 2,3,3,3-tetrafluoropropene and hexafluoropropane is mixed with an extraction solvent to obtain an extraction mixture, and then the obtained extraction mixture is distilled to obtain a distillate having hexafluoropropane as the main component and a distillate including 2,3,3,3-tetrafluoropropene.

Citation List

Patent Literature

Patent Literature 1: JA 2018-002602 A

Summary of Invention

Technical Problem

The present disclosure aims to provide a novel method for separating a fluorocarbon.

Solution to Problem

The present disclosure provides the following aspects.

[1] A method for separating a fluorocarbon gas comprising:

feeding a mixed gas comprising two or more kinds of fluorocarbon gases having different molecular diameters from each other to a porous membrane and separating a gas composition in which a mixing ratio of one kind of a fluorocarbon gas selected from the two or more kinds of fluorocarbon gases is increased or a single gas, wherein the porous membrane comprises amorphous silica formed by a sol-gel method.

[2] The method for separating a fluorocarbon according to [1], wherein the amorphous silica comprises a unit represented by the following formula:

$$-Si-O-Si-$$

[3] The method for separating a fluorocarbon gas according to [1] or [2], wherein the amorphous silica comprises an amorphous silica formed by a sol-gel method using a tetraalkoxysilane as a raw material.
[4] The method for separating a fluorocarbon gas according to any one of [1] to [3], wherein the average pore diameter of porous membrane is 2 Å or more and 10 Å or less.
[5] The method for separating a fluorocarbon gas according to any one of [1] to [4], wherein the one kind of a fluorocarbon gas has a molecular diameter of 2 Å or more and 7 Å or less.
[6] The method for separating a fluorocarbon gas according to any one of [1] to [5], wherein a difference between the molecular diameter of the one kind of a fluorocarbon gas and molecular diameters of other fluorocarbon gases is 0.1 Å or more and 3 Å or less.
[7] The method for separating a fluorocarbon gas according to any one of [1] to [6], wherein the mixed gas is fed to the porous membrane at 20°C or more and 300°C or less.
[8] The method for separating a fluorocarbon gas according to any one of [1] to [7], wherein the mixed gas is fed to the porous membrane at a differential pressure of 0.1 MPa or more.
[9] The method for separating a fluorocarbon gas according to any one of [1] to [8], wherein the porous membrane further comprises a porous support.
[10] The method for separating a fluorocarbon gas according to any one of [1] to [9], wherein the mixed fluorocarbon gas comprises difluoromethane and pentafluoroethane.

[11] The method for separating a fluorocarbon gas according to any one of [1] to [10], wherein feeding of the mixed gas to the porous membrane comprises permeation of at least a part of the mixed gas through the porous membrane.

[12] A separation apparatus, comprising:

a porous membrane,
wherein the porous membrane comprises amorphous silica, formed by a sol-gel method, for separating a gas composition in which a mixing ratio of one kind of a fluorocarbon gas is increased; or a single gas, from a mixed gas comprising two or more kinds of fluorocarbon gases with different molecular diameters.

Advantageous Effects of Invention

According to the present invention, a novel method for separating a fluorocarbon can be provided.

Brief Description of Drawing

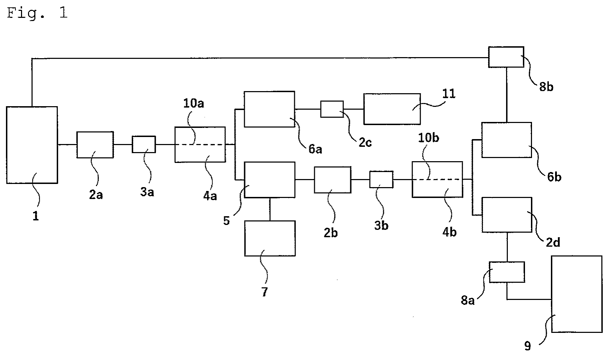

[Figure 1] Figure 1 is a schematic drawing showing a separation apparatus of an embodiment of the present disclosure.

Description of Embodiments

(First embodiment: Method for separating a gas)

The method for separating a gas of the present disclosure comprises feeding a mixed gas comprising two or more kinds of fluorocarbon gases with different molecular diameters from each other to a porous membrane, and separating one kind of a fluorocarbon gas selected from the two or more kinds of fluorocarbon gases,
wherein the porous membrane comprises amorphous silica formed by a sol-gel method.

The present disclosure provides a novel method for separating a fluorocarbon and a separation apparatus. Fluorocarbons have been used as refrigerants and the like in the form of a mixed gas, and the separation by distillation has been attempted. However, the distillation is energy-intensive and azeotropy and the like further complicates the separation process. Furthermore, the differences in the diameters of various fluorocarbons are small, whereby efficient separation and recycling was not easy even by membrane separation.

Known methods for separating a fluorocarbon include, for example, a method that uses a silicone resin as described in JP 1-42444 A and a method that uses a fluorine-based resin as described in Journal of Membrane Science 652 (2022) 120467. However, these methods achieve the separation by utilizing solubility and diffusibility effects in which a gas to be separated dissolves into a membrane, and thus the pore of a separation medium is negligible. The separation method according to the present disclosure differs from the conventional separation methods with respect to allowing fluorocarbon gases to permeate through pores of amorphous silica, which is the separation medium, and achieving the gas separation by the molecular sieve effect. For this reason, the present disclosure provides a novel method for separating a fluorocarbon.

(Mixed gas)

The mixed gas comprises two or more kinds of fluorocarbon gases, and the two or more kinds of fluorocarbon gases have different molecular diameters from each other. Fluorocarbon gases may be representatively a gaseous (aerious) fluorocarbon at 1 atm and room temperature (25°C).

The fluorocarbons are compounds comprising carbon atoms and fluorine atoms bonding to the carbon atoms, and the examples representatively include hydrochlorofluorocarbons, hydrofluorocarbons, and hydrofluoroolefins.

Examples of the hydrochlorofluorocarbon include chlorodifluoromethane and chlorotrifluoroethane.

The examples of the hydrofluorocarbon include hydrofluorocarbons having 1 carbon atom such as difluoromethane; and hydrofluorocarbons having 2 carbon atoms such as difluoroethanes (for example, 1,1-difluoroethane, 1,2-difluoroethane, particularly 1,1-difluoroethane), trifluoroethanes (for example, 1,1,1-trifluoroethane, 1,1,2-trifluoroethane, particularly 1,1,1-trifluoroethane), tetrafluoroethanes (for example, 1,1,1,2-tetrafluoroethane, 1,1,2,2-tetrafluoroethane, particularly 1,1,1,2-tetrafluoroethane), and pentafluoroethane; hydrofluorocarbons having 3 carbon atoms such as tetrafluoropropene and hexafluoropropane.

The examples of hydrofluoroolefin include 2,3,3,3-tetrafluoropropene (HFO-1234yf), 1,3,3,3-tetrafluoropropene (HFO-1234ze), cis-1,1,1,4,4,4-hexafluoro-2-butene (HFO-1336mzz-Z; DR-2), and trans-1,1,1,4,4,4-hexafluoro-2-butene (HFO-1336mzz-E).

The number of carbon atoms of the fluorocarbons may be preferably 1 to 5, and more preferably 1 to 3.

In the fluorocarbons comprised in the mixed gas, the content rate of the fluorocarbons having 1 to 5 carbon atoms may be, relative to the fluorocarbon total amount, for example, 80 mol% or more and 100 mol% or less, and 90 mol% or more and 100 mol% or less.

The two or more kinds of fluorocarbon gases may comprise, for example, a fluorocarbon having 1 carbon atom and a fluorocarbon having 2 carbon atoms, preferably may comprise difluoromethane and pentafluoroethane, and is more preferably composed of difluoromethane and pentafluoroethane. In another aspect, the two or more kinds of fluorocarbon gases may comprise a fluorocarbon having 1 carbon atom and a fluorocarbon having 3 carbon atoms, may comprise preferably difluoromethane (R32) and tetrafluoropropenes (particularly, 2,3,3,3-tetrafluoropropene and R1234yf), and are more preferably composed of difluoromethane (R32) and tetrafluoropropenes (particularly, 2,3,3,3-tetrafluoropropene and R1234yf).

The two or more kinds of fluorocarbon gases have different molecular diameters from each other.

In an aspect, the molecular diameter of one kind of a fluorocarbon comprised in the mixed gas is, for example, 3 Å or more and 6 Å or less, and preferably 3.2 Å or more and 4.4 Å or less. The molecular diameter of one kind of a fluorocarbon comprised in the mixed gas may be, for example, 3 Å or more, and preferably 3.2 Å or more, and for example, 6 Å or less, and preferably 4.4 Å or less.

In the same aspect, the difference in the molecular diameter between one kind of a fluorocarbon and other fluorocarbons comprised in the mixed gas may be preferably 0.1 Å or more, more preferably 0.2 Å or more and 3 Å or less, and further preferably 0.3 Å or more and 2 Å or less.

The molecular diameter of the fluorocarbon gas, given that the shape of fluorocarbon molecules is hard sphere, may be the value calculated by the following equation.

$$d = (ma/(3 \times 2^{1/2} \eta \pi))^{1/2} \cdot \cdot \cdot (x1)$$

wherein, in the equation (x1), d represents the molecular diameter, m represents the mass of one molecule, a represents the molecular speed, and $\eta$ represents the viscosity coefficient.

a can be calculated by the following equation.

$$a = (\gamma RT/M)^{1/2} \cdot \cdot \cdot (x2)$$

$\gamma$ herein is the heat capacity ratio and approximates 1.333. R is the gas constant, T is the absolute temperature, and M is the molar molecular mass.

$\eta$ may be measured by the capillary method.

The molecular diameter of fluorocarbons comprised in the mixed gas may be preferably 2 Å or more and 10 Å or less, and more preferably 3 Å or more and 8 Å or less.

The permeation amount of one kind of a fluorocarbon comprised in the mixed gas through amorphous silica may be preferably $10^{-10}$ (mol/(m$^2$·s·Pa) or more and $10^{-4}$ (mol/(m$^2$·s·Pa) or less, more preferably $10^{-9}$ (mol/ (m$^2$·s·Pa) or more and $10^{-5}$ (mol/(m$^2$·s·Pa) or less, and further preferably $10^{-8}$ (mol/(m$^2$·s·Pa) or more and $10^{-6}$ (mol/(m$^2$·s·Pa) or less.

The boiling point of fluorocarbons comprised in the mixed gas at 1 atm may be, for example, preferably -80°C or more and -20°C or less, more preferably -60°C or more and -30°C or less, and further preferably -50°C or more and -30°C or less. The difference in the boiling points of other fluorocarbons and one kind of a fluorocarbon comprised in the mixed gas at 1 atm may be, for example, preferably 0°C or more and 20°C or less, and more preferably 0°C or more and 10°C or less.

The mixed gas may be an azeotropic mixture or a pseudo-azeotropic mixture of fluorocarbons. According to the method for separating gas of the present disclosure, a specific fluorocarbon can be separated, regardless of an azeotropic mixture or a pseudo-azeotropic mixture. The examples of the azeotropic mixture or pseudo-azeotropic mixture include mixtures having the difference between the boiling point of a fluorocarbon to be separated and the boiling points of other fluorocarbons of 1°C or more and 20°C or less, and further 3°C or more and 18°C or less.

In the mixed gas, the content rate of fluorocarbons relative to the mixed gas total amount may be, for example, 80 mol% or more and 100 mol% or less, and 90 mol% or more and 100 mol% or less.

The mixed gas may comprise other compounds than fluorocarbons. The boiling points of such other compounds may be, for example, -100°C or less, and may be -150°C or less. Such other compounds are representatively comprised in the mixture in the form of an air (gas).

The examples of the above other compounds comprise nitrogen, oxygen, carbon dioxide, and water.

In an aspect, the method for separating gas of the present disclosure may comprise feeding a mixed gas of difluoromethane and pentafluoroethane to a porous membrane, and separating difluoromethane.

(Porous membrane)

The porous membrane is a membrane with a plurality of pores and comprises an amorphous silica formed by a sol-gel method (hereinafter, also referred to simply as "amorphous silica"). The amorphous silica formed by a sol-gel method may be representatively a porous amorphous silica membrane formed by a sol-gel method.

The amorphous silica may comprise a unit represented by a formula: -Si-O-Si- (a siloxane unit), and may further comprise an organic group bonding to the Si atom of the siloxane unit.

In an aspect, the amorphous silica formed by a sol-gel method has a large proportion of the siloxane unit (-Si-O-Si-). In such an aspect, for example, the proportion of the silicon atom to which an organic group is bonded, on the basis of the total amount of the silicon atom comprised in the amorphous silica, may be preferably 0 mol% or more and 20 mol% or less, more preferably 0 mol% or more and 10 mol% or less, and further preferably 0 mol% or more and 5 mol% or less.

In such an aspect, the proportion of the silicon atom represented by $Si(O^{1/2})_4$ (that is, a silicon atom to which 4 oxygen atoms are bonded), on the basis of the total amount of the silicon atom comprised in the amorphous silica formed by a sol-gel method, may be preferably 80 mol% or more and 100 mol% or less, more preferably 90 mol% or more and 100 mol% or less, and further preferably 95 mol% or more and 100 mol% or less.

In another aspect, the amorphous silica formed by a sol-gel method may comprise a siloxane unit (-Si-O-Si-unit) and an organic group bonding to the Si atom of the siloxane unit, and may preferably comprise the siloxane unit and a hydrocarbon group bonding to the Si atom of the siloxane unit.

In such an aspect, for example, the proportion of the silicon atom to which an organic group is bonded, on the basis of the total amount of the silicon atom comprised in the amorphous silica, may be preferably 1 mol% or more and 50 mol% or less, more preferably 1 mol% or more and 20 mol% or less, and further preferably 1 mol% or more and 10 mol% or less.

In such an aspect, the proportion of the silicon atom represented by $Si(O^{1/2})_4$ (that is, a silicon atom to which 4 oxygen atoms are bonded), on the basis of the total amount of the silicon atom comprised in the amorphous silica formed by a sol-gel method, may be preferably 50 mol% or more and 99 mol% or less, more preferably 80 mol% or more and 99 mol% or less, and further preferably 90 mol% or more and 99 mol% or less.

In the present disclosure, the "organic group" means a monovalent or a divalent or higher group comprising carbon or a silsesquioxane group. The organic group may be a hydrocarbon group or a derivative thereof, or a silsesquioxane group, unless otherwise stated. The derivative of a hydrocarbon group may be a group having 1 or more N, O, S, Si, amide, sulfonyl, siloxane, carbonyl, carbonyloxy and the like in the end or a molecular chain of the hydrocarbon group.

In the present disclosure, the "hydrocarbon group" means a monovalent or a divalent or higher group comprising carbon or hydrogen, and a group in which one or more hydrogen atoms are eliminated from the hydrocarbon. Such a hydrocarbon group is not limited, and the examples include $C_{1-20}$ hydrocarbon groups such as aliphatic hydrocarbon group and aromatic hydrocarbon group. The above "aliphatic hydrocarbon group" may be a linear chain, a branched chain or a cyclic chain, and may be saturated or unsaturated. The hydrocarbon group may comprise one or more cyclic structures. The hydrocarbon group may be substituted with 1 or more substituents.

In the case of being used in the present description, the substituent of the "hydrocarbon group" is not limited, and the examples include one or more groups selected from a halogen atom, and a $C_{1-6}$ alkyl group, a $C_{2-6}$ alkenyl group, a $C_{2-6}$ alkynyl group, a $C_{3-10}$ cycloalkyl group, a $C_{3-10}$ unsaturated cycloalkyl group, a 5- to 10-membered heterocyclyl group, a 5- to 10-membered unsaturated heterocyclyl group, a $C_{6-10}$ aryl group, and a 5- to 10-membered heteroaryl group, all of which are optionally substituted with 1 or more halogen atoms.

The average pore diameter of pores of the amorphous silica may be preferably 2 Å or more and 10 Å or less, more preferably 3 Å or more and 8 Å or less, and further preferably 4 Å or more and 6 Å or less. When an average pore diameter of pores of amorphous silica is within such ranges, the separation efficiency of a fluorocarbon may be enhanced.

The average pore diameter of pores of the amorphous silica can be calculated based on the permeability when two or more kinds of gases with different molecular diameters from each other (hydrogen, nitrogen, carbon dioxide, tetrafluoromethane, sulfur hexafluoride and the like) are allowed to permeate. More specifically, two or more kinds of gases with different molecular diameters are measured for the permeation amount respectively, the gas molecular diameter on the abscissa axis and the permeation amount of each gas on the ordinate axis are plotted, and the gas molecular diameter at which a permeation amount is $10^{-7}$ (mol/(m$^2$·s·Pa)) when connecting each plot with a line segment is defined as the average pore diameter (effective pore diameter) of the amorphous silica. The gas molecular diameter may be a kinetic molecular diameter, and as examples, 2.9 Å for a hydrogen gas, 3.6 Å for a nitrogen gas, 4.7 Å for a tetrafluoromethane gas, and 5.1 Å for sulfur hexafluoride. The kinetic molecular diameter may be the value calculated by the above equation (x1).

The amorphous silica thickness of the porous membrane may be preferably 1 nm or more and 1,000 nm or less, more preferably 5 nm or more and 500 nm or less, and further preferably 10 nm or more and 300 nm or less.

The porous membrane comprises amorphous silica, and may further comprise a porous support. In such an aspect, the porous membrane may comprise a porous support and amorphous silica disposed on the porous support, and may preferably comprise a porous support and a porous amorphous silica membrane disposed on the porous support.

The porous membrane, when comprising a porous support, may enhance the stability of the porous membrane. When the mixed gas is fed to the porous membrane, the permeability of the mixed gas through the porous membrane may be enhanced, whereby the separation efficiency is expected to be more enhanced.

The porous support may be either an inorganic porous support or an organic porous support.

The inorganic porous support may be formed of inorganic materials such as silica, alumina, zirconia, silicon carbide, silicon nitride, and mixtures of these. The inorganic porous support may be more preferably a porous alumina support.

The organic porous support may be preferably formed of a heat resistant polymer. The examples of the heat resistant polymer include polysulfone, polyether sulfone, sulfonated polysulfone, sulfonated polyether sulfone, polyimide, polytetrafluoroethylene, polyvinylidene fluoride, and derivatives of these. In the present disclosure, the heat resistant polymer may be understood as a polymer having a glass transition temperature of 100°C or more, and may be preferably understood as a polymer having a glass transition temperature of 200°C or more.

The average pore diameter of pores of the porous support may be preferably 10 nm or more and 500 nm or less, more preferably 15 nm or more and 300 nm or less, and further preferably 20 nm or more and 200 nm or less.

The porous membrane may further comprise a porous intermediate layer between the porous support and the amorphous silica. When such a porous intermediate layer is comprised, the continuity of pore diameters of the amorphous silica and the porous support may be enhanced, whereby the permeability of the mixed gas through porous membrane is expected to be more enhanced.

The porous intermediate layer may be formed of either an inorganic material or an organic material, and is preferably formed of an inorganic material. The examples of the inorganic material include silica, alumina, zirconia, silicon carbide, silicon nitride, and mixtures of these. The organic material is preferably a heat resistant polymer material, and the examples specifically include polysulfone, polyether sulfone, sulfonated polysulfone, sulfonated polyether sulfone, polyimide, polytetrafluoroethylene, polyvinylidene fluoride, and derivatives thereof.

The average pore diameter of pores of the porous intermediate layer may be preferably 0.5 nm or more and 30 nm or less, more preferably 0.7 nm or more and 20 nm or less, and further preferably 1 nm or more and 10 nm or less.

In the present disclosure, when the porous membrane comprises the porous support or the intermediate layer, the average pore diameter of the porous membrane means the average pore diameter of pores of the amorphous silica, and the pore diameter of the porous support or the intermediate layer is irrelevant when measuring and calculating the average pore diameter of the porous membrane.

Such a porous membrane comprises amorphous silica formed by the sol-gel method.

The sol-gel method is a method for producing amorphous silica, using a silane compound having a hydrolyzable group (hereinafter referred to simply as "silane compound") as a raw material, by hydrolyzing and polycondensing the silane compound. In the sol-gel method, the hydrolyzation and polycondensation of individual silane compound form amorphous silica, whereby an organic group (hydrocarbon group) is considered likely to remain in the backbone. For this reason, controlling the pore diameter, polarity, and hydrophobic and hydrophilic properties of the amorphous silica is considered easy. Additionally, the sol-gel method has a feature of being free from the necessity of large equipment compared to vapor deposition methods such as chemical vapor deposition (CVD method).

The above sol-gel method can be carried out by the method comprising the following (a) to (c):

(a) a silane compound having a hydrolyzable group and an aqueous medium are mixed to prepare a silica sol
(b) the silica sol is used to prepare a precursor film
(c) the precursor film is heated to obtain a porous membrane that comprises amorphous silica.

(a) Preparation of the silica sol

In Step (a), a silane compound having a hydrolyzable group and an aqueous medium are mixed to prepare a silica sol. When the silane compound having a hydrolyzable group and the aqueous medium are mixed, the silane compound is hydrolyzed and polycondensed, thereby obtaining the silica sol comprising a silica polymer (oligomer or polymer).

The silane compound having a hydrolyzable group may be representatively a compound that has one or more silicon atoms and a hydrolyzable group bonding to the silicon atom, and may also be a compound having a silicon atom, a hydrolyzable group bonding to the silicon atom, and an organic group bonding to the silicon atom.

The organic group has the same meaning as the organic group comprised in amorphous silica. The hydrolyzable group may be one or more kinds selected from a $C_{1-10}$ alkoxy group, a halogen atom, a hydrogen atom and a hydroxyl group, and preferably a $C_{1-10}$ alkoxy group. The $C_{1-10}$ alkoxy group is preferably a $C_{1-4}$ alkoxy group, more preferably a $C_{1-3}$ alkoxy group, and may be specifically a methoxy group, an ethoxy group or a propoxy group.

The examples of the silane compound having a hydrolyzable group include a compound represented by the following formula:

$$Si(X^1)_n(R^1)_{4-n}$$

wherein

$X^1$ represents a monovalent organic group,
$R^1$ represents each independently at each occurrence a hydrolyzable group,
n represents an integer of 0 to 3, and a compound represented by the following formula:

$$(R_2)_3Si\text{-}X^2\text{-}Si(R^2)_3$$

wherein
$X^2$ represents a divalent organic group,
$R^2$ represents each independently at each occurrence a hydrolyzable group.

The examples of the monovalent organic group represented by $X^1$ include a monovalent hydrocarbon group or a derivative thereof, and a silsesquioxane group. The examples of the monovalent hydrocarbon group include preferably $C_{1-20}$ hydrocarbon groups such as a $C_{1-20}$ aliphatic hydrocarbon group, and a $C_{6-20}$ aromatic hydrocarbon group. The aliphatic hydrocarbon group may be a linear chain, a branched chain, or a cyclic chain, and may be saturated or unsaturated. The hydrocarbon group may comprise one or more cyclic structures. The monovalent hydrocarbon group may be substituted with one or more substituents. The examples of the derivative of the monovalent hydrocarbon group include $C_{3-10}$ heterocyclic groups such as a dioxanyl group, a triazolyl group, a pyridinyl group, a pyradinyl group, a triazinyl group, and an oxalylurea group, a combination group of the $C_{3-10}$ heterocyclic group and a $C_{1-10}$ alkylene group, and a combination group of the $C_{3-10}$ heterocyclic group, an ether bond, and a $C_{1-10}$ alkylene group. The silsesquioxane group may be a polyhedral oligomeric or ladder silsesquioxane group.

The examples of the substituent of the "hydrocarbon group" include one or more groups selected from a halogen atom, and a $C_{1-6}$ alkyl group, a $C_{2-6}$ alkenyl group, a $C_{2-6}$ alkynyl group, a $C_{3-10}$ cycloalkyl group, a $C_{3-10}$ unsaturated cycloalkyl group, a 5- to 10-membered heterocyclyl group, a 5- to 10-membered unsaturated heterocyclyl group, a $C_{6-10}$ aryl group, and a 5- to 10-membered heteroaryl group, an acetoxy group, an amino group, and a hydroxy group, all of which are optionally substituted with one or more halogen atoms.

The organic group represented by $X^1$ is preferably a linear aliphatic hydrocarbon group optionally having a substituent, more preferably a $C_{1-20}$ linear aliphatic hydrocarbon group optionally having a substituent, further preferably a $C_{1-10}$ linear aliphatic hydrocarbon group, and may be specifically alkanediyl groups such as a methyl group, an ethyl group, a butyl group, a hexyl group, and an octyl group; alkenyl groups such as an ethenyl group, alkynyl groups such as an ethynyl group, more preferably a methyl group or an ethyl group. When the number of carbon atoms of X1 is small, the organic group is considered less likely to bend and the pore diameter of the amorphous silica to be obtained is considered likely uniform.

The examples of the divalent organic group represented by $X^2$ comprise a divalent hydrocarbon group or a derivative thereof, and a silsesquioxane group. The examples of the divalent hydrocarbon group preferably include $C_{1-20}$ hydrocarbon groups such as a $C_{1-20}$ aliphatic hydrocarbon group and a $C_{6-20}$ aromatic hydrocarbon group. The aliphatic hydrocarbon group may be a linear chain, a branched chain, or a cyclic chain, and may be saturated or unsaturated. The hydrocarbon group may comprise one or more cyclic structures. The divalent hydrocarbon group may be substituted with one or more substituents. The examples of the derivative of the divalent hydrocarbon group include $C_{3-10}$ heterocyclic groups such as a dioxanediyl group, a triazolylene group, a pyridinylene group, a pyradinylylene group, a triazinylene group, and an oxalylurea group, a combination group of the $C_{3-10}$ heterocyclic group and a $C_{1-10}$ alkylene group, and a combination group of the $C_{3-10}$ heterocyclic group, an ether bond, and a $C_{1-10}$ alkylene group. The silsesquioxane group may be a polyhedral oligomeric or ladder silsesquioxane group.

The examples of the substituent of the "hydrocarbon group" include one or more groups selected from a halogen atom, and a $C_{1-6}$ alkyl group, a $C_{2-6}$ alkenyl group, a $C_{2-6}$ alkynyl group, a $C_{3-10}$ cycloalkyl group, a $C_{3-10}$ unsaturated cycloalkyl group, a 5- to 10-membered heterocyclyl group, a 5- to 10-membered unsaturated heterocyclyl group, a $C_{6-10}$ aryl group, and a 5- to 10-membered heteroaryl group, all of which are optionally substituted with one or more halogen atoms.

The organic group represented by $X^2$ is an aliphatic hydrocarbon group optionally having a substituent, and preferably a linear aliphatic hydrocarbon group. Specifically, the organic group represented by $X^2$ is more preferably a

$C_{1-20}$ linear aliphatic hydrocarbon group optionally having a substituent, further preferably a $C_{2-10}$ linear aliphatic hydrocarbon group, and particularly preferably a linear hydrocarbon group. Specifically, the organic group represented by $X^4$ may be alkanediyl groups such as an ethylene group, a butanediyl group, a hexanediyl group, an octanediyl group; alkenediyl groups such as ethenediyl group, alkynediyl groups such as an acetylenediyl group, and more preferably an ethylene group. When the number of carbon atoms of $X^2$ is small, the organic group is considered less likely to bend and the pore diameter of the amorphous silica to be obtained is considered likely uniform.

The above hydrolyzable group represented by $R^1$ may be one or more kinds selected from a $C_{1-10}$ alkoxy group, a halogen atom, a hydrogen atom and a hydroxyl group, and preferably a $C_{1-10}$ alkoxy group. The $C_{1-10}$ alkoxy group is preferably a $C_{1-4}$ alkoxy group, more preferably a $C_{1-3}$ alkoxy group, and may be specifically a methoxy group, an ethoxy group or a propoxy group.

The hydrolyzable group represented by $R^2$ may be one or more kinds selected from a $C_{1-10}$ alkoxy group, a halogen atom, a hydrogen atom and a hydroxyl group, and preferably a $C_{1-10}$ alkoxy group. The $C_{1-10}$ alkoxy group is preferably a $C_{1-4}$ alkoxy group, more preferably a $C_{1-3}$ alkoxy group, and may be specifically a methoxy group, an ethoxy group or a propoxy group.

The n is an integer of 0 to 3, and preferably an integer of 0 to 1, in an aspect, more preferably 0, and in another aspect, more preferably 1.

The examples of the silane compound represented by $Si(X^1)_n(R^1)_{4-n}$ include tetraalkoxysilanes such as tetramethoxysilane, tetraethoxysilane, and tetrapropoxysilane; and alkylalkoxysilanes such as methyltrimethoxysilane, methyltriethoxysilane, ethyltrimethoxysilane, ethyltriethoxysilane, vinyltrimethoxysilane, and vinyltriethoxysilane.

The examples of the compound represented by $(R_2)_3SiX^2\text{-}Si(R^2)_3$ include bis triethoxysilyl ethane, bis triethoxysilyl butane, bis triethoxysilyl octane, bis triethoxysilyl ethylene, and bis triethoxysilyl acetylene.

The examples of the aqueous medium include water, and a mixture of water and a hydrophilic solvent. Examples of the hydrophilic solvent include alcohols such as methanol, ethanol, propanol.

The method for mixing the silane compound and the aqueous medium is not limited, and is representatively carried out by stirring the mixture of the silane compound and the aqueous medium.

When mixing the silane compound and the aqueous medium, a catalyst may be allowed to be present therewith. The catalyst may facilitate the hydrolyzation and polycondensation of the silane compound. For such a catalyst, acid catalysts such as hydrochloric acid, nitric acid, and acetic acid; and base catalysts such as ammonia may be used.

(b) Preparation of the precursor film

In Step (b), a precursor film is prepared using the silica sol. In the present disclosure, the precursor film comprises a silica polymer (oligomer or polymer) and may comprise a part of the aqueous medium.

The preparation of the precursor film may be representatively carried out by applying the silica sol obtained in Step (a). When preparing the porous membrane comprising the porous support and/or porous intermediate layer, the silica sol may be applied onto the porous support and/or porous intermediate layer.

The examples of the method for applying the silica sol include spin coating, dip coating, and a method in which a nonwoven fabric is immersed in the silica sol and applied, but are not limited thereto.

After application of the silica sol, representatively, the aqueous medium is dried to prepare the precursor film. The drying can be carried out at room temperature.

(c) Heating

In Step (c), the precursor film is heated to obtain a porous membrane comprising amorphous silica. The heating of the precursor film may facilitate the hydrolyzation and polycondensation of a silica polymer (oligomer or polymer) in the silica sol thereby densely forming siloxane bonds, whereby amorphous silica is formed.

The heating temperature may be preferably 100°C or more and 400°C or less, and more preferably 100°C or more and 200°C or less. The heating time may be preferably 10 minutes or more and 60 minutes or less, and more preferably 15 minutes or more and 30 minutes or less. The heating under these heating conditions facilitates the formation of pores in amorphous silica, and when an organosilane compound having an organic group is used as the silane compound, the organic group can be retained.

When producing the porous membrane, Step (b) and Step (c) may be repeatedly carried out twice or more. When Step (b) and Step (c) are repeated twice or more, the silica sol in Step (b) may be applied onto the amorphous silica formed in the previous Step (c).

(Contact method)

When the mixed gas comprising fluorocarbons is fed to the porous membrane, at least one kind of a fluorocarbon permeates through pores of the porous membrane thereby separating such a fluorocarbon. Fluorocarbons may be representatively fed to the porous membrane in the form of a gas (air).

In an aspect, according to the separation method, the porous membrane selectively allows only a specific fluorocarbon to permeate, whereby the specific fluorocarbon may be separated from the mixed gas comprising two or more kinds of fluorocarbons. In a preferable aspect, according to the separation method, a fluorocarbon having 1 carbon atom may be separated from the mixed gas comprising two or more kinds of fluorocarbons with different molecular diameters.

The feeding of the mixed gas to the porous membrane may be carried out by, for example, the column method. The column method can be carried out by fixing the porous membrane on a container (column) and flowing fluorocarbons through the container (column).

In an aspect, the temperature at the feeding may be, for example, 10°C or more and 40°C or less, and may further be 15°C or more and 35°C or less. The pressure (gauge pressure) at the feeding may be 0.2 MPaG or more and 2 MPaG or less, and may be 0.4 MPaG or more and 1.5 MPaG or less. When a pressure at the feeding is within the above range, fluorocarbons easily permeate through the porous membrane, whereby the separation efficiency may be enhanced and the pore structure of the porous membrane may be retained.

The flow rate of the mixed gas may be 0.001 kg/hr or more and 1,000 kg/hr or less, 0.005 kg/hr or more and 500 kg/hr or less, and further 0.01 kg/hr or more and 100 kg/hr.

Only the fluorocarbons permeated through the porous membrane are recovered, thereby recovering the specific fluorocarbons. In this aspect, the method for separating a fluorocarbon gas of the present disclosure may also be understood as the method for recovering a fluorocarbon gas.

(Second embodiment: Composite material)

The composite material comprising the porous membrane and fluorocarbons is also encompassed in the technical scope of the present disclosure. When the mixed gas is fed to the porous membrane, at least a part of the fluorocarbons comprised in the mixed gas remains in the porous membrane, whereby the composite material can be produced.

The fluorocarbons and the porous membrane in the present embodiment have the same meaning as the fluorocarbons and the porous membrane in the first embodiment.

In the composite material, the fluorocarbons preferably comprise a fluorocarbon having 1 carbon atom, and may more preferably comprise one or more kinds selected from chlorodifluoromethane and difluoromethane.

In a preferable aspect, the content of a fluorocarbon having 1 carbon atom in the composite material is larger than the content of fluorocarbons having 2 or more carbon atoms, and more preferably the total content of one or more kinds selected from chlorodifluoromethane and difluoromethane is larger than the content of fluorocarbons other than those.

The composite material may comprise a resin other than the fluorocarbons and the porous membrane. The examples of the resin include an acrylic resin, a polyurethane resin, a polyolefin resin, a polyester resin, a polyamide resin, a vinyl chloride resin, a styrene resin, a vinyl ether resin, a polyvinyl alcohol resin, a polycarbonate resin and a polysulfone resin.

The composite material may also comprise additives such as an emulsifier, an antifoaming agent, a surfactant, a leveling agent, a thickener, a viscoelastic modifier, an antifoaming agent, a wetting agent, a dispersant, a preservative, a plasticizer, a penetrating agent, a flavor, a disinfectant, a miticide, a fungicide, an UV absorber, an antioxidant, an antistatic agent, a flame retardant, a dye, and a pigment.

(Third embodiment: Separation apparatus)

Hereinafter, the separation apparatus in an embodiment of the present disclosure will be described, but the present disclosure is not limited to such an aspect.

The apparatus of the present disclosure comprises

a porous membrane,
wherein the porous membrane comprises amorphous silica, for separating a gas composition in which a mixing ratio of one kind of a fluorocarbon gas is increased; or a single gas, from a mixed gas comprising two or more kinds of fluorocarbon gases with different molecular diameters.

In Figure 1, a 2-staged separation apparatus will be schematically described as an example of the separation apparatus of the present disclosure.

In the separation apparatus of Figure 1, a mixed gas comprising two or more kinds of fluorocarbon gases with different molecular diameters is stored in a gas tank 1. The mixed gas is fed to a separation module 4a of the first stage via a pressure regulator 2a and a mass flow controller 3a. In an aspect, the pressure regulator 2a may regulate the pressure, but is not limited to the aspect. The pressure regulator 2a can be optionally left out.

The separation module 4a comprises a porous membrane 10a, and the mixed gas is fed to the porous membrane 10a. During this feeding, the pressure may also be regulated by a nonpermeation-side back pressure regulating valve 6a. Representatively, the pressure may be reduced by the nonpermeation-side back pressure regulating valve 6a. The nonpermeation-side back pressure regulating valve 6a can be optionally left out.

The gasses that did not permeate the porous membrane 10a (hereinafter, also referred to as "the first nonpermeation-side gas") are recovered in a nonpermeation-side gas recovery pipe 11 via a pressure regulator 2c. The first nonpermeation-side gases may be recovered in the gas tank 1. In an aspect, the flow rate of the first nonpermeation-side gasses may be regulated by the mass flow controller.

In the 2-staged separation apparatus, the gasses that permeated the porous membrane 10a (hereinafter, also referred to as "the first permeated gases") are measured for a permeation amount by a mass flow meter 5a and fed to a separation module 4b of the second stage via the pressure regulator 2b and the mass flow controller 3b. In an aspect, the pressure may be regulated by the pressure regulator 2b, but is not limited to the aspect. The pressure regulator 2b can be optionally left out.

The separation module 4b of the second stage comprises a porous membrane 10b, and the first permeated gases are fed to the porous membrane 10b. During this feeding, the pressure may also be regulated by a nonpermeation-side gas back pressure regulating valve 6b. Representatively, the pressure may be reduced by the nonpermeation-side back pressure regulating valve 6a. The nonpermeation-side gas back pressure regulating valve 6b can be optionally left out.

Of the first permeated gasses, the gases that did not permeate through the porous membrane 10b (hereinafter, also referred to as "the second nonpermeation-side gas") is analyzed for the gas composition with a gas composition analyzer 8b and then allowed to circulate to the gas tank 1. In another aspect, the second nonpermeation-side gas may be recovered separately by the nonpermeation-side gas recovery pipe. In another aspect, the second nonpermeation-side gas may be allowed to circulate separately between the mass flow meter 5 and the pressure regulator 2b. In an aspect, the gas composition analyzer 8b can be optionally left out.

Of the first permeated gases, the gases that permeated through the porous membrane 10b (hereinafter, also referred to as "the second permeated gases") is analyzed for the gas composition with a gas composition analyzer 8a via a pressure regulator 2d and then recovered in a recovery pipe 9. In an aspect, the permeation amount of a gas permeated through the porous membrane 10b may be measured using a mass flow meter. In an aspect, the gas composition analyzer 8a can be optionally left out.

In the present embodiment, the 2-staged separation apparatus has been described, but suitable changes can be made without being limited thereto and, for example, a 1-staged, a 3-staged or a 4- or more-staged separation apparatus may also be employed. In the case of a 1-staged apparatus, the first permeated gasses may be recovered in the recovery pipe, and the first nonpermeation-side gases may be recovered by the nonpermeation-side gas recovery pipe or may be allowed to circulate to the gas tank 1.

In the case of n-staged apparatus (n is 3 or more), the permeated gases that permeated through the separation module of the n-1 stage are fed to the separation molecule via the pressure regulator and the mass flow controller, and via the porous membrane provided in the separation module, the n-th permeated gasses that permeated the porous membrane and the n-th nonpermeation-side gases that did not permeate the porous membrane may be separated. The n-th permeated gasses, after the gas composition analysis that may be carried out as needed, are recovered, whereby the gas separation can be carried out. The n-th nonpermeation-side gases, after the gas analysis that may be carried out as needed, may be allowed to circulate to the gas tank 1 or recovered in the nonpermeation-side gas recovery pipe.

Examples

The present invention will be described in further details in reference to the following examples, but is not limited thereto.

(Production Examples 1 to 3)

A silane compound having a hydrolyzable group (1,2-bis(triethoxysilyl)ethane) and water were mixed in such a way so that the concentration of the silane compound was 5% by mass and stirred to prepare a silica sol. The silica sol was applied by spin coating method to obtain a precursor film. Under a nitrogen atmosphere, the obtained precursor film was heated at 100°C for 30 minutes. The application and heating repeatedly carried out twice to obtain porous membranes 1 to 3 comprising amorphous silica.

(Examples 1 to 9)

Fluorocarbon gases adjusted to a predetermined pressure and flow rate using a pressure regulator and a mass flow controller (MFC) were allowed to flow through a membrane module comprising an amorphous silica membrane. Using the pressure regulator and the mass flow meter (MFM), the pressure and the flow rate of the permeated side and the nonpermeation-side were confirmed. A pressure reducing pump was connected on the permeated side, and the measurement was carried out under reduced pressure condition as needed. The permeation degree of a single component gas was calculated from the flow rate.

The fluorocarbons used were R32 (difluoromethane) and R125 (1,1,1,2,2-pentafluoroethane). The molecular diameter of R32 is 3.9 Å and the molecular diameter of R125 is 4.4 Å.

The molecular diameter of the fluorocarbons used the values described in Wanigarathna, J. A. D. K. (2018). Adsorption-based fluorocarbon separation in zeolites and metal organic frameworks. Doctoral thesis, Nanyang Technological University, Singapore.

The pore diameter of the amorphous silica membrane was carried out by permeability tests using hydrogen gas, nitrogen gas, tetrafluoromethane gas, and sulfur hexafluoride. Specifically, the permeation amounts were measured in the same manner as in Example 1, in the exception that hydrogen gas (molecular diameter: 2.9 Å), nitrogen gas (molecular diameter: 3.6 Å), tetrafluoromethane gas (molecular diameter: 4.7 Å), and sulfur hexafluoride (molecular diameter: 5.1 Å) were used in place of the fluorocarbon gas. The molecular diameter of each gas was plotted on the abscissa axis, the permeation amount was plotted on the ordinate axis, and each plot was connected with a line segment. In the plots and the line segments, the value of molecular diameter at which the permeation amount was $10^{-7}$ (mol/(m$^2$·s·Pa)) was defined as the pore diameter of each membrane. The molecular diameters of the hydrogen gas, nitrogen gas, tetrafluoromethane gas, and sulfur hexafluoride were kinetic molecular diameters. The kinetic molecular diameter can also be the value calculated by the above equation (x1).

[Table 1]

| | Pore diameter | Temperature | Feeding-side differential pressure | Permeation amount (R32) | Permeation amount (R125) | Permeation amount ratio |
|---|---|---|---|---|---|---|
| | Å | °C | kPaA | mol/(m$^2$ · s · Pa) | | |
| Example 1 | 4.0 | 200 | 100 | $1.84\times10^{-7}$ | $2.62\times10^{-10}$ | 702 |
| Example 2 | 4.0 | 100 | 100 | $1.95\times10^{-7}$ | $2.79\times10^{-10}$ | 699 |
| Example 3 | 4.0 | 30 | 100 | $1.41\times10^{-7}$ | $2.02\times10^{-9}$ | 70 |
| Example 4 | 4.5 | 200 | 100 | $5.66\times10^{-7}$ | $5.07\times10^{-9}$ | 112 |
| Example 5 | 4.5 | 100 | 100 | $6.65\times10^{-7}$ | $5.81\times10^{7}$ | 115 |
| Example 6 | 4.5 | 30 | 100 | $6.62\times10^{7}$ | $6.71\times10^{-9}$ | 99 |
| Example 7 | 5.0 | 200 | 100 | $1.27\times10^{-6}$ | $2.14\times10^{-8}$ | 59 |
| Example 8 | 5.0 | 100 | 100 | $1.49\times10^{-6}$ | $1.53\times10^{-8}$ | 97 |
| Example 9 | 5.0 | 30 | 100 | $1.72\times10^{-6}$ | $8.75\times10^{-9}$ | 197 |

Examples 1 to 9 are the examples of the present disclosure, in which the fluorocarbons were fed to the porous membranes comprising amorphous silica. In all examples, it was confirmed that the permeation amounts of R32 (difluoromethane) were higher than the permeation amounts of R125 (1,1,1,2,2-pentafluoroethane), and the ratios of permeation amounts of R32 to permeation amounts of R125 were 59 to 699 times. These findings reveal that when a mixed gas comprising two or more kinds of fluorocarbon gases is fed to the porous membranes comprising amorphous silica, one kind of fluorocarbon gas selected from the two or more kinds of fluorocarbon gases can be separated.

(Examples 10 and 11: Mixed gas evaluation)

A mixed gas of R32 and R125 adjusted to a predetermined pressure and flow rate using a pressure regulator and a mass flow controller (MFC) was allowed to flow through a membrane module. Using the pressure regulator and the mass flow meter (MFM), the pressure and the flow rate of the permeated side and the nonpermeation-side were confirmed. A pressure reducing pump was connected on the permeated side, and the measurement was carried out under reduced pressure condition as needed. Subsequently, the composition analysis was carried out using GC.

The following table shows the pore diameter of amorphous silica membranes, the composition of the mixed gas (ratio of R32 to R125, the temperature when measured, the feeding side differential pressure and permeated side pressure, permeation amounts (ratio of R32 to R125), and permeation degree ratio.

[Table 2]

| | Pore diameter | Composition (R32/R125) | Temperature | Feeding-side pressu | Permeatedside pressure | Permeation amount (R32) | Permeation amount (R125) | Permeation amount ratio |
|---|---|---|---|---|---|---|---|---|
| | Å | wt% | °C | kPaA | kPaA | mol/(m$^2$ · s · Pa) | | |
| Example 10 | 5.0 | 50/50 | 30 | 200 | 100 | $7.45 \times 10^{-7}$ | $1.00 \times 10^{-8}$ | 74 |
| Example 11 | 5.0 | 85/15 | 30 | 200 | 100 | $1.18 \times 10^{-6}$ | $1.13 \times 10^{-8}$ | 104 |

Examples 10 to 11 are the examples of the present disclosure, in which the mixed gas of the fluorocarbons was fed to the porous membranes comprising amorphous silica. In all examples, it was confirmed that the permeation amounts of R32 (difluoromethane) were higher than the permeation amounts of R125 (1,1,1,2,2-pentafluoroethane), and the ratios of permeation amounts of R32 to permeation amounts of R125 were 59 to 699 times. These findings reveal that when a mixed gas comprising two or more kinds of fluorocarbon gases is fed to the porous membranes comprising amorphous silica, one kind of a fluorocarbon gas selected from the two or more kinds of fluorocarbon gases can be separated.

Reference Signs List

> 1 Gas tank
> 2a, 2b, 2c, 2d Pressure regulator
> 3a, 3b Mass flow controller
> 4a, 4b Separation module
> 5 Mass flow meter
> 6a, 6b Nonpermeation-side back pressure regulating valve
> 7 Pressure reducer and booster
> 8a, 8b Gas composition analyzer
> 9 Separated gas recovery pipe
> 10a, 10b Porous membrane
> 11 Nonpermeation-side gas recovery pipe

## Claims

1. A method for separating a fluorocarbon gas comprising:

   feeding a mixed gas comprising two or more kinds of fluorocarbon gases having different molecular diameters from each other to a porous membrane and separating a gas composition in which a mixing ratio of one kind of a fluorocarbon gas selected from the two or more kinds of fluorocarbon gases is increased or a single gas, wherein the porous membrane comprises amorphous silica formed by a sol-gel method.

2. The method for separating a fluorocarbon according to claim 1, wherein the amorphous silica comprises a unit represented by a formula:

   $$-Si-O-Si-$$

3. The method for separating a fluorocarbon gas according to claim 1 or 2, wherein the amorphous silica comprises an amorphous silica formed by a sol-gel method using a tetraalkoxysilane as a raw material.

4. The method for separating a fluorocarbon gas according to any one of claims 1 to 3, wherein average pore diameter of the porous membrane is 2 Å or more and 10 Å or less.

5. The method for separating a fluorocarbon gas according to any one of claims 1 to 4, wherein the one kind of a fluorocarbon gas has a molecular diameter of 2 Å or more and 7 Å or less.

6. The method for separating a fluorocarbon gas according to any one of claims 1 to 5, wherein a difference between the molecular diameter of the one kind of a fluorocarbon gas and molecular diameters of other fluorocarbon gases is 0.1 Å or more and 3 Å or less.

7. The method for separating a fluorocarbon gas according to any one of claims 1 to 6, wherein the mixed gas is fed to the porous membrane at 20°C or more and 300°C or less.

8. The method for separating a fluorocarbon gas according to any one of claims 1 to 7, wherein the mixed gas is fed to the porous membrane at a differential pressure of 0.1 MPa or more.

9. The method for separating a fluorocarbon gas according to any one of claims 1 to 8, wherein the porous membrane further comprises a porous support.

10. The method for separating a fluorocarbon gas according to any one of claims 1 to 9, wherein the mixed fluorocarbon gas comprises difluoromethane and pentafluoroethane.

11. The method for separating a fluorocarbon gas according to any one of claims 1 to 10, wherein feeding of the mixed gas to the porous membrane comprises permeation of at least a part of the mixed gas through the porous membrane.

12. A separation apparatus, comprising:

a porous membrane,
wherein the porous membrane comprises an amorphous silica, formed by a sol-gel method, for separating a gas composition in which a mixing ratio of one kind of a fluorocarbon gas is increased; or a single gas, from a mixed gas comprising two or more kinds of fluorocarbon gases with different molecular diameters.

Fig. 1

## INTERNATIONAL SEARCH REPORT

| International application No. |
|---|
| **PCT/JP2024/012254** |

**A. CLASSIFICATION OF SUBJECT MATTER**

*B01D 53/22*(2006.01)i; *B01D 61/58*(2006.01)i; *B01D 69/00*(2006.01)i; *B01D 69/10*(2006.01)i; *B01D 69/12*(2006.01)i; *B01D 71/70*(2006.01)i; *C07C 17/389*(2006.01)i; *C07C 19/08*(2006.01)i
FI: B01D53/22; B01D61/58; B01D69/00; B01D69/10; B01D69/12; B01D71/70 500; C07C17/389; C07C19/08

According to International Patent Classification (IPC) or to both national classification and IPC

**B. FIELDS SEARCHED**

Minimum documentation searched (classification system followed by classification symbols)

B01D53/22; B01D61/00-71/82; C02F1/44; C07B31/00-61/00; C07B63/00-63/04; C07C1/00-409/44

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2017-023966 A (NIPPON SHOKUBAI CO., LTD.) 02 February 2017 (2017-02-02) claims, examples | 1-12 |
| A | JP 2018-143931 A (HIROSHIMA UNIVERSITY) 20 September 2018 (2018-09-20) claims, examples | 1-12 |
| A | JP 2001-062241 A (KYOCERA CORPORATION) 13 March 2001 (2001-03-13) claims, examples | 1-12 |
| A | JP 2011-526882 A (THE SOUTH AFRICAN NUCLEAR ENERGY CORPORATION LIMITED) 20 October 2011 (2011-10-20) claims | 1-12 |

☐ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **04 June 2024** | **25 June 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**
Information on patent family members

International application No.

**PCT/JP2024/012254**

| Patent document cited in search report | | | Publication date (day/month/year) | Patent family member(s) | | | Publication date (day/month/year) |
|---|---|---|---|---|---|---|---|
| JP | 2017-023966 | A | 02 February 2017 | (Family: none) | | | |
| JP | 2018-143931 | A | 20 September 2018 | (Family: none) | | | |
| JP | 2001-062241 | A | 13 March 2001 | (Family: none) | | | |
| JP | 2011-526882 | A | 20 October 2011 | US claims | 2011/0094377 | A1 | |
| | | | | WO | 2010/001368 | A1 | |
| | | | | EP | 2318121 | A1 | |
| | | | | CN | 102123780 | A | |
| | | | | KR | 10-2011-0059591 | A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018002602 A

- JP 1042444 A

**Non-patent literature cited in the description**

- *Journal of Membrane Science*, 2022, vol. 652, 120467

- Adsorption-based fluorocarbon separation in zeolites and metal organic frameworks. **WANIGARATHNA, J. A. D. K.** Doctoral thesis. Nanyang Technological University, 2018